Europäisches Patentamt
European Patent Office
Office européen des brevets

(19)

(11) Publication number: 0 325 936 A2

(12) **EUROPEAN PATENT APPLICATION**

(21) Application number: 89100345.1

(22) Date of filing: 10.01.89

(51) Int. Cl.4: C07C 133/10 , C07D 213/76 , A61K 31/155 , A61K 31/44

Claims for the following Contracting States: ES + GR.

(30) Priority: 16.01.88 JP 5721/88

(43) Date of publication of application: 02.08.89 Bulletin 89/31

(84) Designated Contracting States: AT BE CH DE ES FR GB GR IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Ohuchida, Shuichi Ono Pharmaceutical Co., Ltd.
Minase Research Insti. 3-1-1 Sakurai
Shimamoto-cho
Mishimagun Osaka(JP)
Inventor: Toda, Masaaki Ono Pharmaceutical Co., Ltd.
Minase Research Insti. 3-1-1 Sakurai
Shimamoto-cho
Mishimagun Osaka(JP)
Inventor: Miyamoto, Tsumoru Ono Pharmaceutical Co., Ltd.
Minase Research Insti. 3-1-1 Sakurai
Shimamoto-cho
Mishimagun Osaka(JP)

(74) Representative: Henkel, Feiler, Hänzel & Partner
Möhlstrasse 37
D-8000 München 80(DE)

(54) **Aminoguanidine derivatives and inhibitory agents on maillard reaction containing them as active ingredients.**

(57) An aminoguanidine derivative of the general formula:

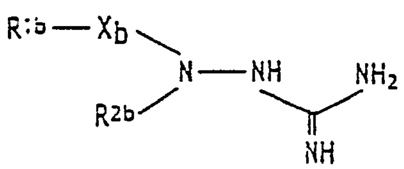

(IB)

(wherein, $R^{1b}$ represents carbocyclic or heterocyclic ring substituted or unsubstituted by from 1 to 3 group(s) selected from halogen atom, alkyl or alkoxy group of from 1 to 4 carbon atom(s), nitro group, phenoxy group, amino group, hydroxy group and acylamino group of from 2 to 4 carbon atom(s), $X_b$ represents single-bond, alkylene group of from 1 to 4 carbon atom(s) or alkenylene group of from 2 to 4 carbon atoms, or $R^{1b}$ together with $X_b$ represents alkyl group of from 1 to 4 carbon atom(s), $R^{2b}$ represents hydrogen atom, alkyl group of from 1 to 4 carbon atom(s) or phenyl group substituted or unsubstituted by from 1 to 3 group(s) selected from halogen atom, alkyl or alkoxy group of from 1 to 4 carbon atom(s), hydroxy and nitro group.), or an acid addition salt thereof possesses inhibitory activity on Maillard reaction, and these is useful for prevention and/or treatment of several diabetic complications and deseases induced by aging.

EP 0 325 936 A2

# Aminoguanidine derivatives and inhibitory agents on Maillard reaction containing them as active ingredients.

## Summary

This invention is related to aminoguanidine derivatives and medicines containing them as active ingredients.

More particularly, this invention is related to

(1) the compounds of the general formula:

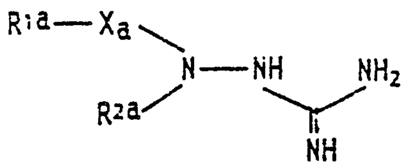

(IA)

(wherein, all of the symbols represent the same meaning as hereinafter defined.),

(2) the process for the preparation of the compounds of the general formula (IA) and

(3) inhibitory agents on Maillard reaction containing the compounds of the general formula:

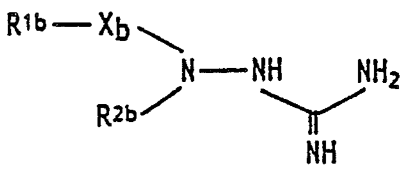

(IB)

(wherein, all of the symbols represent the same meaning as hereinafter defined.), which includes the compounds of the formula (IA), as active ingredient.

## [Background]

In 1912, Maillard reported that, when a mixture of amino acid and reducing sugar is heated, it shows brown color (called browning phenomenon). [Maillard, L.C., Compt. Rend. Soc. Biol., 72, 599 (1912)], and suggested that this reaction might occur in a body.

In 1968, Rahbar reported that HbAlc, the glycosylated part of haemoglobin (Hb), was increased in diabetics [Rahbar, S., Clin, Chim. Acta., 22, 296 (1968)].

Afterwards, it became clean that HbAlc had the chemical structure in which glucose binds to valine in N-terminal side of $\beta$-chain in the form of Amadori rearrangement [Koenig, R.J., Blobstein, S.H., & Cerami, A., J. Biol. Chem., 252, 2992 (1977)], and that Maillard reaction proceeds nonenzymatically [Stevens, V.J., Vlassara, H., Abati, A., & Cerami, A., J. Biol. Chem., 252, 2998 (1977)]. These results showed that Maillard reaction occurs in a body.

As an initial step, Maillard reaction consists of forming the Amadori rearrangement products by glycosylation of reducing sugar with amino-group of protein. In the advanced stage of this reaction

① cross-linked compounds [called advanced glycosylation products (abbreviated as "AGE")] are produced,

② solubility of them becomes low,

③ the products can not be easily degraded by the action of proteases,

④ a fluorescent is formed, and then

⑤ the products are colored brown.

The mechanism of AGE production has been proposed by some groups. For example, the theory of Brownlee et al is shown below [Brownlee, M. et al., Science, 232, 1629 (1986)].

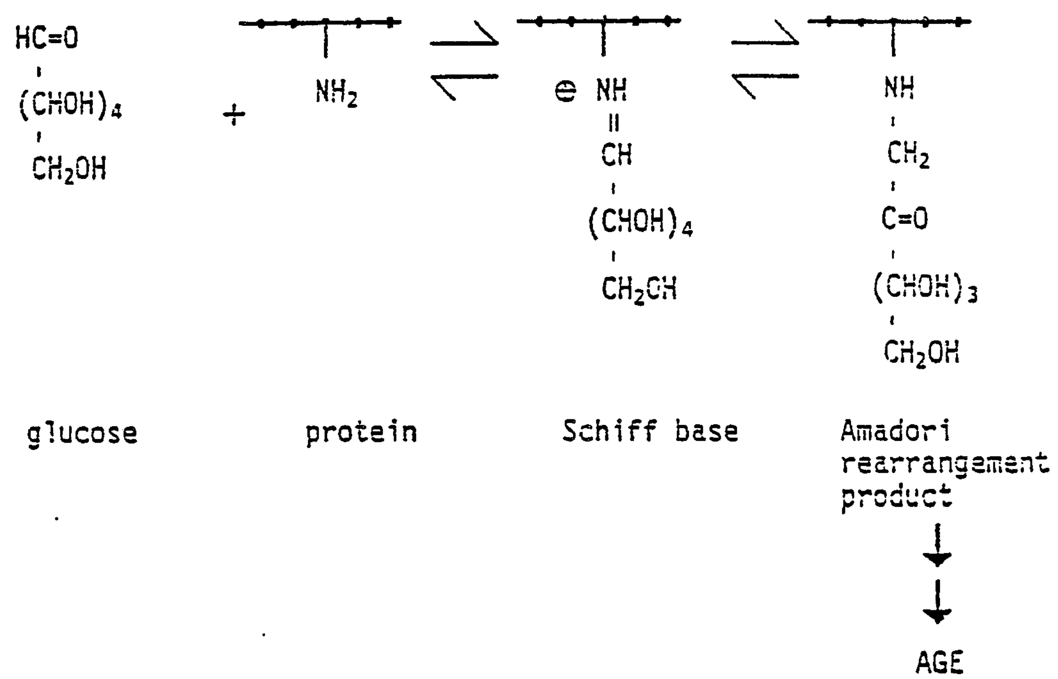

glucose protein Schiff base Amadori rearrangement product AGE

Maillard reaction is observed in healthy person, and particularly this is caused notably in diabetics that a level of blood sugar is high and in protein of which metabolic turnover is slow.

For example, in the case of haemoglobin, the level of glycosylation in diabetic mouse are 2.7 times as high as that of normal mouse [Monnier, V.M. et al., The Maillard Reaction in Foods and Nutrition, ACS Symposium Series, 215, 432, Am. Chem. Soc., Washington, D.C. (1983)], and glycosylation of serum albumin in diabetics was advanced [Guthrow, C.E. et al., Proc. Natl. Acad. Sci. U.S. 76, 4258 (1979)].

Further, it was turned out that, when a glycosylated serum protein was administrated intravenously during 12 weeks, the typical diabetic renal lesion was observed [Monnier, V.M. et al., Clin. Endocrinol. Metab., 11, 431 (1982)].

Crystallin in lens is the special protein which is not metabolized at all after its biosynthesis.
When crystallin was glycosylated, its steric structure was transformed and the SH groups were oxidized to form S-S bond. A sequence of processes induced to polymerize the protein in crystallin.

In the case of diabetic cataract in rats, the ratio of binding of protein and glucose was ten times as high as that of normal rat, and also intramolecular S-S bond formation increased [Monnier, V.M. & Cerami, A. Clin. Endocrinol. Metab. 11, 431 (1982)].

Glycosylation of crystallin caused its polymerization, decrease in its solubility, formation of fluorescent substance, yellow and brown coloring. This phenomenon is very similar to that of lens by aging [Chiou, S.H., Chylack, L. T., Jr., Tung, W.H., & Bunu, F., J. Biol. Chem. 256, 5176 (1981)].

Collagen and elastin in connective tissue contain lysine and hydroxylysine abundantly, the speed of their metabolic turnover is slow, and the existance of reactants with glucose at the basement membrance of renal adrenal glands, skin and tendon has been found [Monnier, V.M., Stevens, V.J., & Cerami, A., Maillard Reactions in Food, Prog. Food Nutr. Sci. 5, 315, Pergamon Press, London], further these glycosylation products might be related to sclerosis in a blood vessel wall [Rosenburg, H., Modrak, J.B., Hassing, J.M., Al-Turk, W.A., & Stohs, S.J., Biochem.Biophys.Res. Commun, 91, 498 (1979)].

The cause of diabetic neurosis may be nonenzymatic glycosylation of neuro-myelin protein [Monnier, V.M. et al., Clin.Endocrinol.Metab. 11, 431 (1982)].

In this way, Maillard reaction in a body is related to not only various diabetic complication but also a lot of diseases accompanied with aging.

[Prior arts]

On the above background, recently, researches of Maillard reaction inhibitors has been carried out. For example, Brownlee, M. et al. showed that aminoguanidine inhibits Maillard reaction in vitro, and that aminoguanidine, administered to diabetic rats, inhibits diabetes-induced accumulation of advanced

glycosylation end products in arterial wall connective tissue protein [Brownlee, M. et al., Science, 232, 1629 (1986)].

They have considered that the amino group of a nucleophilic hydrazine compound (i.e. the amino group which is bonded to guanidino group in aminoguanidine) blocks reactive carbonyls on early glycosylation products, and inhibits further cross-link formation of chemically reversible Amadori product.

Further, in the specification of Japanese Patent Kokai No. 62-142114 i.e. European Patent Publication No. 222313, it was suggested that the pharmaceutical composition comprising compound having the group containing reactive nitrogen atoms, which enable to react with reactive carbonyls in chemically reversible Amadori product, inhibits the production of advanced glycosylation end products. Concretely, the compositions comprising aminoguanidine, α-hydrazino histidine and lysine are disclosed.

And recently, urea, guanidine and salts thereof were disclosed concretely as compositions which inhibit cross-link formation of collagen in the specification of Japanese Patent Kokai No. 62-249908 i.e WPI Accession No.87-345514/49.

And further, as the example of compounds which have an equal or similar structure with those of this invention, in the specification of British Patent No. 1259568, it is suggested that the compounds of the general formula:

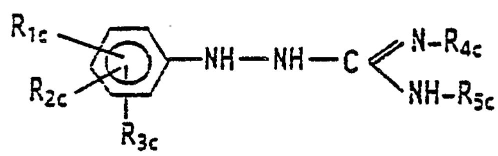

(C)

(wherein, $R_{1c}$ represents nitro group, alkyl, alkoxy, alkylthio or haloalkyl, $R_{2c}$ and $R_{3c}$ each represents the same or different group selected from hydrogen atom, halogen, hydroxy group, alkyl and alkoxy, $R_{4c}$ and $R_{5c}$ each represents the same or different group selected from hydrogen atom and alkyl group of more than 3 carbon atoms). are useful as antihypertensive agents.

In this specification of British Patent, (2,4-dimethylanilino) guanidine, (2,3-dimethylanilino)guanidine, (3,4-dimethylanilino) guanidine, (2, 6-dimethylanilino)guanidine, (2-methyl-6-chloroanilino) guanidine, (2-methyl-3-chloroanilino)guanidine, (2-methyl-4-chloroanilino)guanidine, (3-chloro-4-methylanilino)guanidine, (2-methyl-5-chloroanilino)guanidine, (3-chloro-4-methoxyanilino) guanidine, (2-methoxy-5-chloroanilino)-guanidine, (2-methoxy-4-chloro-5-methylanilino)guanidine, (2-trifluoromethylanilino)guanidine, (3-trifluoromethylanilino)guanidine, (4-nitroanilino)guanidine and (4-methylthioanilino)guanidine are disclosed concretely.

[Disclosure of the invention]

The present invention is related to

1) the compound of the general formula:

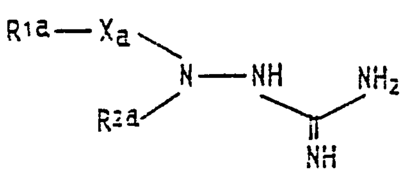

(IA)

(wherein, $R^{1a}$ represents carbocyclic or heterocyclic ring substituted or unsubstituted by from 1 to 3 groups-(s) of halogen atom, alkyl or alkoxy group of from 1 to 4 carbon atom(s), or nitro group, phenoxy group, amino group, hydroxy group or acylamino group from 2 to 4 carbon atoms, $X_a$ represents single-bond, alkylene of from 1 to 4 carbon atoms(s) or alkenylene of from 2 to 4 carbon atom(s), or $R^{1a}$ together with $X_a$, represents alkyl group of from 1 to 4 carbon atom(s), $R^{2a}$ represents hydrogen atom, alkyl group of from 1 to 4 carton atom(s) or phenyl group substituted or unsubstituted by from 1 to 3 group(s) selected from halogen atom, alkyl or alkoxy group of from 1 to 4 carbon atom(s), hydroxy and nitro group.

With the proviso that the compounds wherein the group of $-X_a-R^{1a}$ represents 3-nitrophenyl group, 4-nitrophenyl group, 3-chloro-4-methoxyphenyl group, 2-methoxy-5-chlorophenyl group, 2,4-dimethylphenyl group, 2,3-dimethylphenyl group, 3,4-dimethylphenyl group, 2,6-dimethylphenyl group, 2-methyl-6-chlorophenyl group, 2-methyl-3-chlorophenyl group, 2-methyl-4-chlorophenyl group, 3-chloro-4-methylphenyl group, 2-methyl-5-chlorophenyl group, 2-methoxy-4-chloro-5-methylphenyl group, 2-trifluoromethylphenyl group, 3-trifluoromethylphenyl group or 4-methylthiophenyl group, and further $R^{2a}$ represents hydrogen atom are excluded.) and acid addition salts thereof,

2) A process for the preparation of the aminoguanidine derivatives of the general formula:

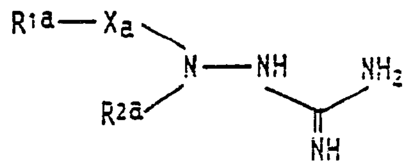

(IA)

(wherein all symbols represent the same meanings as described hereinbefore.)
which is characterized by the general formula:

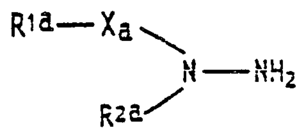

(II)

(wherein all symbols represent the same meaning as described hereinbefore.)
and the compound of the general formula:

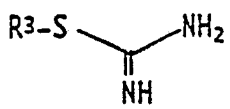

(III)

(wherein the symbols represent the same meaning as described hereinbefore.).

(3) inhibitory agents on Maillard reaction containing the compounds of the general formula:

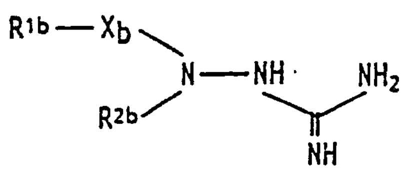

(IB)

(wherein, $R^{1b}$ represents carbocyclic or heterocyclic ring substituted or unsubstituted by from 1 to 3 group(s) selected from halogen atom, alkyl or alkoxy group of from 1 to 4 carbon atom(s), nitro group, phenoxy group, amino group, hydroxy group and acylamino group of from 2 to 4 carbon atom(s), $X_b$ represents single-bond, alkylene group of from 1 to 4 carbon atom(s) or alkenylene group of from 2 to 4 carbon atoms, or $R^{1b}$ together with $X_b$ represents alkyl group of from 1 to 4 carbon atom(s), $R^{2b}$ represents hydrogen atom, alkyl group of from 1 to 4 carbon atom(s) or phenyl group substituted or unsubstituted by from 1 to 3 group(s) selected from halogen atom, alkyl or alkoxy group of from 1 to 4 carbon atom(s), hydroxy and nitro group.), which includes the compounds of formula (IA), or acid addition salts thereof as active ingredients.

[Comparison with prior arts]

It has been considered that the group containing reactive nitrogen atom (i.e. the amino group which is bonded to guanidino group) of aminoguanidine blocks reactive carbonyl groups in early glycosylation

products, and aminoguanidine inhibits Maillard reaction. Accordingly it can be unexpected that the compounds of the present invention in which the amino group is blocked, have an inhibitory action on Maillard reaction.

And a part of the compounds of the present invention is the same structure as the compounds of the general formula (C).

However, it is disclosed that the compounds of the general formula (C) are useful as antihypertensive agents, and it can be unexpected from the efficacy and use of these drug that the compounds of the present invention possess an inhibitory action on Maillard reaction.

The present invention includes all isomers unless otherwise specified.

For example, alkyl group, alkoxy group, alkylene group and alkenylene group mean straight-chain or branched-chain alkyl group, alkoxy group, alkylene group and alkenylene group, and the double-bond in alkenylene group includes E, Z and mixture of E and Z.

And, in the case of existing branched-chain alkyl group, the present invention includes the isomers caused by existing asymmetrical carbon atoms.

In the general formulae (IA) and (IB), halogen atom shown by substituent groups in $R^{1a}$, $R^{1b}$, $R^{2a}$ and $R^{2b}$ are fluorine atom, chlorine atom, bromine atom and iodine atom, acylamino group of from 2 to 4 carbon atoms shown by substituent groups in $R^{1a}$ and $R^{1b}$ are acetoamido, propionamido, butylamido and isomeric groups thereof, and any groups are preferable.

In the general formulae (IA) and (IB), alkyl group of from 1 to 4 carbon atoms shown by substituent groups in $R^{1a}$, $R^{1b}$, $R^{2a}$ and $R^{2b}$, shown by $R^{1a}$ together with $X_a$, shown by $R^{2b}$ together with $X_b$, and shown by $R^{2a}$ and $R^{2b}$ are methyl, ethyl, propyl, butyl and isomeric groups thereof, and alkoxy group of from 1 to 4 carbon atom(s) shown by substituent groups in $R^{1a}$, $R^{1b}$, $R^{2a}$ and $R^{2b}$ are methoxy, ethoxy, propoxy, butoxy and isomeric groups thereof, and any groups are preferable. Preferable substituent groups in $R^{1a}$ and $R^{1b}$ are nitro, phenoxy, amino and hydroxy group. Especially, chlorine atom, fluorine atom, methyl, methoxy and nitro group are preferable, and futher preferably $R^{1a}$ and $R^{1b}$ each represents unsubstituted carbocyclic and heterocyclic rings.

In the general formulae (IA) and (IB), carbocyclic rings in $R^{1a}$ and $R^{1b}$ are mono-, bi- or tri-aromatic carbocyclic rings containing not more than 15 carbon atoms which may be partially or fully saturated.

The rings are, for example, benzene, naphthalene, indene, azulene, fluorene, phenanthrene, anthracene, acenaphthylene, biphenylene ring and the rings which may be partially or fully saturated thereof. Preferable rings are benzene and naphthalene ring.

In the general formulae (IA) and (IB), heterocyclic rings in $R^{1a}$ and $R^{1b}$ are mono-, bi- or tri- aromatic heterocyclic rings containing not more than 15 carbon and hetero atoms which may be partially or fully saturated. For example, they are furan, thiophene, pyrrole, oxazole, isooxazole, thiazole, isothiazole, imidazole, pyrazole, furazan, pyran, pyridine, pyridazine, pyrimidine, pyrazine, indole, isoindole, benzofuran, benzothiophene, indolizine, chromene, quinoline, isoquinoline, quinolizine, purine, indazole, quinazoline, cinnoline, quinoxaline, phthalazine, pteridine, carbazole, acridine, phenanthridine, xanthene, phenazine, phenothiazine rings, and partially or fully saturated rings thereof.

Preferable substituent groups in $R^{2a}$ and $R^{2b}$ are hydroxy and nitro group, preferable $R^{2a}$ and $R^{2b}$ are hydrogen atom and unsaturated phenyl group, especially preferable $R^{2a}$ and $R^{2b}$ are hydrogen atoms, methyl group and phenyl group.

In the general formulae (IA) and (IB), alkylene groups of from 1 to 4 carbon atom(s) shown by $X_a$ and $X_b$ are methylene, ethylene, trimethylene, tetramethylene group and isomeric groups thereof, and alkenylene groups of from 2 to 4 carbon atoms are vinylene, propenylene, butenylene group and isomeric groups thereof, especially preferable $X_a$ and $X_b$ are single-bond.

And the compounds of the general formulae (IA) and (IB), if desired, may be converted into acid addition salts by the known methods.

Preferably, acid addition salts are non-toxic salts and water-soluble.

The suitable acid addition salts are, for example, hydrochloric acid, hydrobromic acid, hydroiodic acid, sulfuric acid, phosphoric acid, an inorganic acid such as nitric acid, or an organic acid such as acetic acid, lactic acid, tartric acid, benzoic acid, citric acid, methanesulphonic acid, ethanesulphonic acid, benzenesulphonic acid, toluenesulphonic acid, isethionic acid, glucuronic acid and gluconic acid.

Acid addition salts may be obtained by the known methods, for example, by reacting stoichiometric quantities of a compound of general formula (IA) or (IB) and the appropriate acid in a suitable solvent.

[Process for the preparation of the compounds of the present invention]

The compounds of the general formula (IA) may be prepared by the step described hereinafter or by the step described in the specification of British Patent No. 1259568.

The compounds of the general formula (IA) may be prepared by reacting the compound of the general formula:

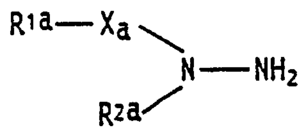

(II)

(wherein, all symbols represent the same meaning as hereinafter defined.) with the compound of the general formula:

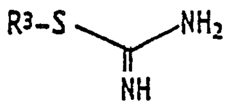

(III)

(wherein, $R^3$ represents alkyl group of from 1 to 4 carbon atom(s).) in water at from room temperature to 80 °C.

The compounds of the general formula (IB) may be also prepared by the same method as the compounds of the general formula (IA).

[Starting materials]

In the present invention, all of material compounds of the general formula (II) and (III) and each reagent are known or may be prepared by known method.

The reaction products may be purified by conventional methods, for example, distillation at atmospheric or reduced pressure, high performance liquid chromatography, thin layer chromatography or column chromatography using silica gel or magnesium silicate or washing or recrystallization. Purification may be carried out after each reactions or a series of reactions.

[Effect]

The compounds of the general formulae (IA) and (IB), of the present invention, and acid addition salts thereof possess the inhibitory activity on Maillard reaction. Accordingly, the compounds of the present invention are useful for treatment and/or prevention of several diabetic complication, i.e. coronary heart disease, peripheral circulatory insufficiency or failure, cerebrovascular hindrance, neurogenous diabetes, nephropathy, arteriosclerosis, arthrosclerosis, cataracta and retinopathy, and the diseases induced by aging, i.e. atherosclerosis, senile cataract and cancer.

An inhibitory activity on Maillard reaction of the compounds of the present invention was confirmed by the screening system mentioned below.

(1) Method of experiment

In order to evaluate the inhibitory effect on Maillard reaction in vitro, of the compounds in the present invention, the measurement was carried out under conditions described below.

100 mg/ml bovine serum albumin (BSA), 200 mM glucose and 6mM test compound were dissolved in 0.5 M phosphatic buffer solution (pH 7.38), and the mixture was incubated for a week at 37 °C.

After incubation, the incubated medium was diluted 100 times with the same phosphatic buffer solution, and the fluorescence spectrum was measured at the excitation maximum of 360nm and the emission maximum of 450 nm.

Inhibition percentage of the test compounds was calculated by the following equation:

$$\text{Inhibition (\%)} = \frac{\Delta I - \{\Delta I_4 - (\Delta I_1 + \Delta I_2 + \Delta I_3)\}}{\Delta I}$$

$\Delta I_1$ : fluorescence of test compounds
$\Delta I_2$ : fluorescence of (test compounds + glucose),
$\Delta I_3$ : fluorescence of (test compounds + BSA),
$\Delta I_4$ : fluorescence of (test compounds + BSA + glucose),
$\Delta I$ : fluorescence of (BSA + glucose)

(2) Results

The results are shown in the following table I.

Table 1

| Example No. | $-X_a-R^{1a}$ or $-X_b-R^{1b}$ | $R^{2a}$ or $R^{2b}$ | Salts | Name | Inhibitory effect (%) (6mM) |
|---|---|---|---|---|---|
| 1 | 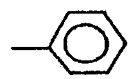 | H | Sulfate | 1-anilinoguanidine sulfate | 79 |
| 1(a) | 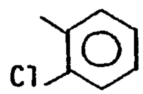 | H | Sulfate | 1-(2-chloroanilino) guanidine sulfate | 79 |
| 1(b) | 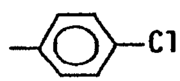 | H | Sulfate | 1-(4-chloroanilino) guanidine sulfate | 100 |
| 1(c) | 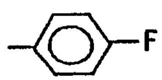 | H | Sulfate | 1-(4-fluoroanilino) guanidine sulfate | 100 |
| 1(d) | 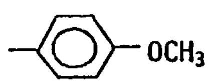 | H | Sulfate | 1-(4-methoxyanilino) guanidine sulfate | 100 |
| 1(h) | 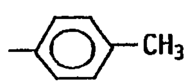 | H | Sulfate | 1-(4-methylanilino) guanidine sulfate | 83 |
| 1(j) | 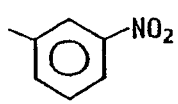 | H | Sulfate | 1-(3-nitroanilino) guanidine sulfate | 94 |

The results in the table I show that the compounds of the present invention and acid addition salts possess inhibitory effect on Maillard reaction.

[Toxicity]

It was confirmed that the toxicity of the compounds of the present invention were very low.

Accordingly it was confirmed that the compounds of the present invention were useful for prevention and/or treatment for deseases attributed to Maillard reaction, in animals including human beings, especially human beings.

[Administration]

For the purpose above described, the compounds of the present invention of the general formula (IA), (IB) and acid addition salts thereof may normally by administered systemically or partially, usually by oral or parenteral administration.

The doses to be administered are determined depending upon age, body weight, symptom, the desired therapeutic effect, the route of administration, and the duration of the treatment etc. In the human adult, the doses per person per dose are generally between 1 mg and 1000 mg and 100 mg, by parenteral administration (preferably, intravenous administration) up to several times per day.

As mentioned above, the doses to be used depend upon various conditions. Therefore, there are cases in which doses lower than or greater than the ranges specified above may be used.

Solid compositions for oral administration in the present invention, include compressed tablets, pills, dispersible powders, and granules. In such compositions, one or more of the active compound(s) is or are, admixed with at least one inert diluent (hydroxypropylcellulose, microcrystalline cellulose, starch, polyvinylpyrrolidone, magnesium metasilicate aluminate etc.). The compositions may also comprise, as is normal practice, additional substances other than inert diluents: e.g. lubricating agents (magnesium stearate etc.), disintegrating agents (cellulose calcium gluconate etc.), stabilizing agent (lactose etc.), and assisting agent for dissolving (glutamic acid, aspertic acid etc.).

The tablets or pills may, if desired, be coated with film of gastric or enteric material (sugar, gelatin, hydroxypropylcellulose or hydroxypropylmethyl cellulose phthalate etc.), or be coated with more than two films. And further, it may be include capsules of absorbable materials such as gelatin.

Liquid compositions for oral administration include pharmaceutically-acceptable solutions, emulsions, suspensions, syrups and elixirs.

In such compositions, one or more of the active compound(s) is or are comprise in inert diluent(s) commonly used in the art (purified water, ethanol etc.).

Besides inert diluents, such compositions may also comprise adjuvants (wetting agents, suspending agents etc.), sweetening agents, flavouring agents, perfuming agents and preserving agents.

Other compositions for oral administration include spray compositions which may be prepared by known methods and which comprise one or more of the active compound(s).

Spray compositions may comprise additional substances other than inert diluents: e.g. stabilizing agents (sodium sulfite etc.), isotonic buffer (sodium chloride, sodium citrate, citric acid etc.).

For preparation of such spray compositions, for example, the method described in the United States Patnet No. 2868691 or 3095355 may be used.

Injections for parenteral administration include sterile aqueous or non-aqueous solutions, suspensions and emulsions. In such compositions, one more of active compound(s) is or are admixed at least one of inert aqueous diluent(s) (distilled water for injection, physiological salt solution etc.) or inert non-aqueous diluent(s) (propylene glycol, polyethylene glycol, olive oil, ethanol, POLYSOLBATE 80 (registered trade mark) etc.).

Injections may comprise additional other than inert diluents: e.g. preserving agents, wetting agents, emulsifying agents, dispersing agents, stabilizing agents (lactose etc.), assisting agents such as assisting agents for dissolving (glutamic acid, aspertic acid etc.).

They may be sterillized, for example, by filtration through a bacteria-retaining filter, by incorporation of sterilizing agents in the compositions or by irradiation. They also be manufactured in the form of sterile solid compositions, for example, by freeze-drying, and which can be dissolved in sterile water or some other sterile diluents for injection immediately before used.

Other compositions for parenteral administration include liquids for external use, and endermic linimetns (ointment etc.), suppositories and pessaries which comprise one or more of the active compound(s) and may be prepared by known methods.

[Example]

The following examples illustrate the compounds of the present invention and the process for the preparation of them, but not limit the present invention.

The solvents in the parentheses show the developing or eluting solvents and the rations of the solvents used are by volume in chromatographic separations. "IR" were measured by KBr tablet method.

Example 1

1-anilinoguanidine sulfate

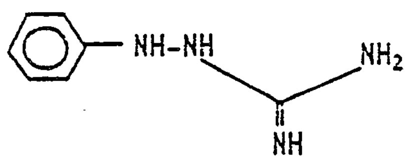

$\cdot 1/2\ H_2SO_4$

Phenylhydrazine (1.83 ml) was added to an aqueous solution (10 ml) of S-methylisothiourea sulfate (2.0 g). The mixture was stirred for 3 hours at 80° C, and the reaction mixture was allowed to cool to room temperature and concentrated under reduced pressure. The residue was dissolved in hot ethanol and the insoluble material was removed by filtration. The filtrate was concentrated, and the crystal was given. The crystal was recrystallized from water to purify. The title compound having the following physical data was given (698 mg).

TLC: Rf 0.41 (ethyl acetate : acetic acid : water = 3 : 1 : 1);

IR: $\nu$ 3100, 1640, 1590, 1490, 1100, 750, 610 $cm^{-1}$.

Example 1(a) ~ 1(l)

The compounds of the present invention shown in the following table II were obtained with using the compound of the general formula:

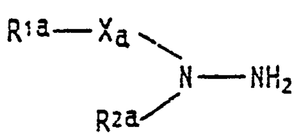

(II)

(wherein, all of the symbols represent the same meaning as hereinbefore defined) as the starting materials, by the same procedure as example 1.

Table II

| Example No. | | Name | TLC | IR |
|---|---|---|---|---|
| 1(a) | 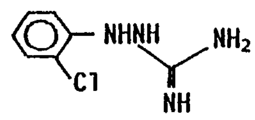 ·1/2 $H_2SO_4$ | 1-(2-chloroanilino) guanidine sulfate | Rf 0.82 (ethyl acetate: acetic acid: water = 3 : 1 : 1) | ν 3600~2400, 1685, 1625, 1490, 1105 $cm^{-1}$ |
| 1(b) | 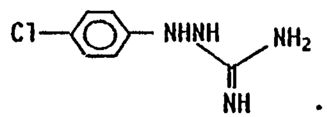 ·1/2 $H_2SO_4$ | 1-(4-chloroanilino) guanidine sulfate | Rf 0.37 (ethyl acetate: acetic acid: water = 30 : 3 : 1) | ν 3600~2500, 3450, 3140, 2850, 1660, 1580, 1485, 1100 $cm^{-1}$ |
| 1(c) | 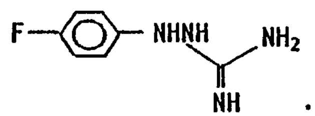 ·1/2 $H_2SO_4$ | 1-(4-fluoroanilino) guanidine sulfate | Rf 0.35 (ethyl acetate: acetic acid: water = 30 : 3 : 1) | ν 3600~2500, 3480, 3370, 3140, 2850, 1665, 1600, 1505, 1200 $cm^{-1}$ |
| 1(d) | 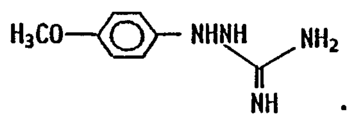 ·1/2 $H_2SO_4$ | 1-(4-methoxyanilino) guanidine sulfate | Rf 0.16 (ethyl acetate: acetic acid: water = 30 : 3 : 2) | ν 3600~2500, 3370, 3100, 1660, 1590, 1505, 1240, 1140~1030 $cm^{-1}$ |
| 1(e) | 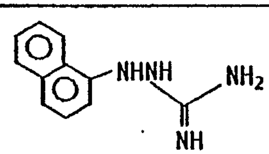 ·1/2 $H_2SO_4$ | 1-(1-naphthylamino) guanidine sulfate | Rf 0.30 (ethyl acetate: acetic acid: water = 30 : 3 : 2) | ν 3500~2600, 3460, 1670, 1610, 1395, 1100, 1045 $cm^{-1}$ |
| 1(f) | 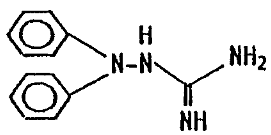 ·1/2 $H_2SO_4$ | 1-(N,N-diphenylamino) guanidine sulfate | Rf 0.22 (chloroform: methanol = 20 : 1 ) | ν 3510, 3400~2700, 2900, 1690, 1585, 1480, 1265, 750 $cm^{-1}$ |

Table II (continue)

| Example No. | | Name | TLC | IR |
|---|---|---|---|---|
| 1(g) | 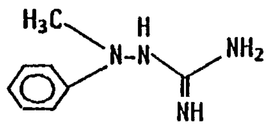 $\cdot 1/2\ H_2SO_4$ | 1-(N-methyl-N-phenylamino) guanidine sulfate | Rf 0.18 (chloroform: methanol = 20 : 1) | ν 3400~3315, 3210, 3500~2600, 1660, 1595, 1460, 1390 $cm^{-1}$ |
| 1(h) | 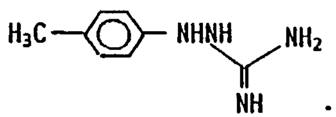 $\cdot 1/2\ H_2SO_4$ | 1-(4-methylanilino) guanidine sulfate | Rf 0.15 (ethyl acetate: acetic acid: water = 30 : 3 : 2) | ν 3650~2500, 1640, 1490, 1250, 1110, 815 $cm^{-1}$ |
| 1(i) | 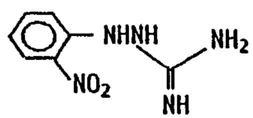 $\cdot 2\ CH_3COOH$ | 1-(2-nitroanilino) guanidine acetate | Rf 0.33 (ethyl acetate: acetic acid: water = 30 : 3 : 2) | ν 3600~2100, 3340, 1680~1590, 1520, 1400, 1350, 1280, 1140 $cm^{-1}$ |
| 1(j) | 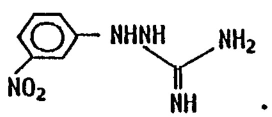 $\cdot 1/2\ H_2SO_4$ | 1-(3-nitroanilino) guanidine sulfate | Rf 0.14 (ethyl acetate: acetic acid: water = 30 : 3 : 2) | ν 3600~2600, 3460, 3410, 3200, 1675, 1635, 1520, 1350, 1110 $cm^{-1}$ |
| 1(k) | 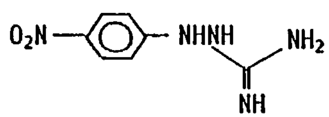 $\cdot 2\ CH_3COOH$ | 1-(4-nitronilino) guanidine acetate | Rf 0.21 (ethyl acetate: acetic acid: water = 30 : 3 : 2) | ν 3600~2200, 3450, 1680, 1595, 1480, 1410, 1335 1110 $cm^{-1}$ |
| 1(l) | 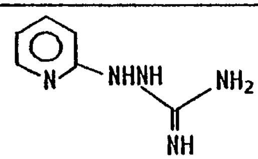 $\cdot H_2SO_4$ | 1-(2-pyridylamino) guanidino sulfate | Rf 0.47 (chloroform : THF : ethyl acetate = 30 : 3 : 2) | ν 3500~2300, 1680, 1600, 1430, 1050, 960 $cm^{-1}$ |

[Preparative example]

The following components were admixed in conventional method and punched out to obtain 100 tablets each containing 50 mg of active ingredient.

| | |
|---|---|
| ° 1-anilinoguanidine sulfate .... | 5 g |
| ° Cellulose Calcium gluconate (disintegrating agent) .... | 0.2 g |
| ° Magnesium stearate (lubricating agent) .... | 0.1 g |
| ° Microcrystaline cellulose .... | 4.7 g |

## Claims

1) An aminoguanidine derivative of the general formula:

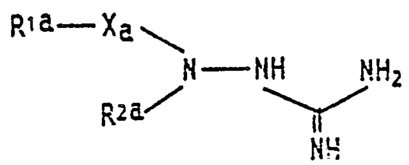

(IA)

(wherein, $R^{1a}$ represents carbocyclic or heterocyclic ring substituted or unsubstituted by from 1 to 3 group(s) selected from halogen atom, alkyl or alkoxy group of from 1 to 4 carbon atom(s), nitro group, phenoxy group, amino group, hydroxy group and acylamino group of from 2 to 4 carbon atoms,
$X_a$ represents single-bond, alkylene of from 1 to 4 carbon atom(s) or alkenylene of from 2 to 4 carbon atom(s), or $R^{1a}$ together with $X_a$, represents alkyl group of from 1 to 4 carbon atom(s), $R^{2a}$ represents hydrogen atom, alkyl group of from 1 to 4 carton atom(s) or phenyl group substituted or unsubstituted by from 1 to 3 group(s) selected from halogen atom, alkyl or alkoxy group of from 1 to 4 carbon atom(s), hydroxy and nitro group.
With the proviso that the compounds wherein the group of $-X_a-R^{1a}$ represents 3-nitrophenyl group, 4-nitrophenyl group, 3-chloro-4-methoxyphenyl group, 2-methoxy-5-chlorophenyl group, 2,4-dimethylphenyl group, 2,3-dimethylphenyl group,. 3,4-dimethylphenyl group, 2,6-dimethylphenyl group, 2-methyl-6-chlorophenyl group, 2-methyl-3-chlorophenyl group, 2-methyl-4-chlorophenyl group, 3-chloro-4-methylphenyl group, 2-methyl-5-chlorophenyl group, 2-methoxy-4-chloro-5-methylphenyl group, 2-trifluoromethylphenyl group, 3-trifluoromethylphenyl group or 4-methylthiophenyl group, and further $R^{2a}$ represents hydrogen atom are excluded.) and acid addition salts thereof,

2) A compound according to claim 1, wherein $X_a$ represents single-bond.

3) A compound according to claim 2, wherein carbocyclic ring in $R^{1a}$ represents benzene or naphthalene.

4) A compound according to claim 1, wherein $R^{1a}$ represents carbocyclic or heterocyclic ring substituted or unsubstituted by chlorine atom, fluorine atom, methyl group, methoxy group or nitro group.

5) A compound according to claim 1, wherein $R^{2a}$ represents hydrogen atom, methyl group or phenyl group.

6) A compound according to claim 3, which is
1-(4-chloroanilino)guanidine,
1-(4-fluoroanilino)guanidine,
1-(4-methoxyanilino)guanidine or
1-(4-methylanilino)guanidine.

7) A process for the preparation of the aminoguanidine derivatives of the general formula:

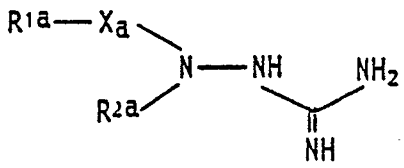

(IA)

(wherein all symbols represent the same meanings as described hereinbefore.) which is characterized by reacting the compound of the general formula:

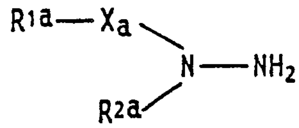

(II)

(wherein all symbols represent the same meaning as described hereinbefore.) and the compound of the general formula:

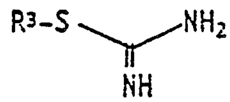

(III)

(wherein $R^3$ represents alkyl group of from 1 to 4 carbon atom(s).).

8) A pharmaceutical composition, which comprises the administration of an effective amount of the aminoguanidine derivative of the general formula:

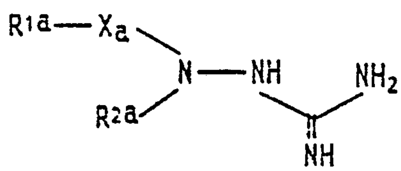

(IA)

(wherein, $R^{1a}$ represents carbocyclic or heterocyclic ring substituted or unsubstituted by from 1 to 3 groups(s) selected from halogen atom, alkyl or alkoxy group of from 1 to 4 carbon atom(s), nitro group, phenoxy group, amino group, hydroxy group and acylamino group of from 2 to 4 carbon atoms,

$X_a$ represents single-bond, alkylene of from 1 to 4 carbon atom(s) or alkenylene of from 2 to 4 carbon atom(s), or $R^{1a}$ together with $X_a$, represents alkyl group of from 1 to 4 carbon atom(s), $R^{2a}$ represents hydrogen atom, alkyl group of from 1 to 4 carbon atom(s) or phenyl group substituted or unsubstituted by from 1 to 3 group(s) selected from halogen atom, alkyl or alkoxy group of from 1 to 4 carbon atom(s), hydroxy and nitro group.

With the proviso that the compounds wherein the group of $-X_a-R^{1a}$ represents 3-nitrophenyl group, 4-nitrophenyl group, 3-chloro-4-methoxyphenyl group, 2-methoxy-5-chlorophenyl group, 2,4-dimethylphenyl group, 2,3-dimethylphenyl group, 3,4-dimethylphenyl group, 2,6-dimethylphenyl group, 2-methyl-6-chlorophenyl group, 2-methyl-3-chlorophenyl group, 2-methyl-4-chlorophenyl group, 3-chloro-4-methylphenyl group, 2-methyl-5-chlorophenyl group, 2-methoxy-4-chloro-5-methylphenyl group, 2-trifluoromethylphenyl group, 3-trifluoromethylphenyl group or 4-methylthiophenyl group, and further $R^{2a}$ represents hydrogen atom are excluded.) and acid addition salts thereof,

9) For use in the prevention and/or treatment of several diabetic complications and diseases induced by aging, a compound of the aminoguanidine derivatives of the general formula (IA).

10) An aminoguanidine derivative of the general formula (IB), wherein the group of $-X_b-R^{1b}$ represents 3-nitrophenyl group, 4-nitrophenyl group, 3-chloro-4-methoxyphenyl group, 2-methoxy-5-chlorophenyl group, 2,4-dimethylphenyl group, 2,3-dimethylphenyl group, 3,4-dimethylphenyl group, 2,6-dimethylphenyl group, 2-methyl-6-chlorophenyl group, 2-methyl-3-chlorophenyl group, 2-methyl-4-chlorophenyl group, 3-chloro-4-methylphenyl group, 2-methyl-5-chlorophenyl group, 2-methoxy-4-chloro-5-methylphenyl group, 2-trifluoromethylphenyl group, 3-trifluoromethylphenyl group or 4-methylthiophenyl group, and further $R^{2b}$ represents hydrogen atom, or an acid addition salts thereof for use in the manufacture of a medicament.

11) 1-(3-nitroanilino)guanidine or 1-(4-nitroanilino)guanidine or an acid addition salts thereof for use in the manufacture of a medicament.

Claim for the following Contracting States: ES, GR

A process for the preparation of the aminoguanidine derivatives of the general formula:

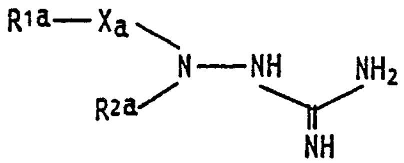

(IA)

(wherein, $R^{1a}$ represents carbocyclic or heterocyclic ring substituted or unsubstituted by from 1 to 3 group-(s) of halogen atom, alkyl or alkoxy group of from 1 to 4 carbon atom(s), nitro group, phenoxy group, amino group, hydroxy group or acylamino group from 2 to 4 carbon atoms, $X_a$ represents single-bond, alkylene of from 1 to 4 carbon atom(s) or alkenylene of from 2 to 4 carbon atom(s), $R^{1a}$ together with $X_a$, represents alkyl group of from 1 to 4 carbon atom(s), $R^{2a}$ represents hydrogen atom, alkyl group of from 1 to 4 carton atom(s) or halogen atom, phenyl group substituted or unsubstituted by one, two or three group(s) of alkyl or alkoxy group of from 1 to 4 carbon atom(s), hydrogen or nitro group.

With the proviso that the compounds wherein the group of -Xa-R1a represents 3-nitrophenyl group, 4-nitrophenyl group,3-chloro-4-methoxyphenyl group, 2-methoxy-5-chlorophenyl group, 2,4-dimethylphenyl group, 2,3-dimethylphenyl group, 3,4-dimethylphenyl group, 2,6-dimethylphenyl group, 2-methyl-6-chlorophenyl group, 2-methyl-3-chlorophenyl group, 2-methyl-4-chlorophenyl group, 3-chloro-4-methylphenyl group, 2-methyl-5-chlorophenyl group, 2-methoxy-4-chloro-5-methylphenyl group, 2-trifluoromethylphenyl group, 3-trifluoromethylphenyl group and 4-methylthiophenyl group, and further $R^{2a}$ represents hydrogen atom are excluded.)

which is characterized by reacting the compound of the general formula:

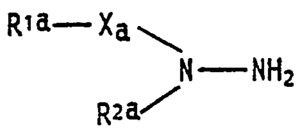

(II)

(wherein all symbols represent the same meaning as described hereinbefore.)

and the compound of the general formula:

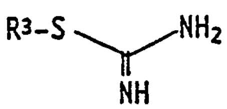

(III)

(wherein $R^3$ represents alkyl group of from 1 to 4 carbon atom(s).).